# EUROPÄISCHE PATENTSCHRIFT

(11) **EP 1 656 334 B1**
(45) Veröffentlichungstag und Bekanntmachung des Hinweises auf die Patenterteilung: **22.08.2007**
(21) Anmeldenummer: 04763397.9
(22) Anmeldetag: 22.07.2004
(51) Int. Cl.: C07C 7/08

(54) **VERFAHREN ZUR AUFTRENNUNG EINES ROH-C4-SCHNITTES**
METHOD FOR THE SEPARATION OF A CRUDE C4 CUT
PROCEDE POUR DECOMPOSER UNE COUPE C SB 4 /SB BRUTE

(30) Priorität: 24.07.2003 DE 10333756
(43) Veröffentlichungstag der Anmeldung: 17.05.2006
(73) Patentinhaber: BASF Aktiengesellschaft, 67056 Ludwigshafen (DE)
(72) Erfinder: HEIDA, Bernd, 67158 Ellerstadt (DE)
(74) Vertreter: Isenbruck, Günter
(86) Internationale Anmeldenummer: PCT/EP2004/008192
(87) Internationale Veröffentlichungsnummer: WO 2005/009931

(56) Entgegenhaltungen:
- EP-A- 0 284 971
- WO-A-20/04011406
- DE-A- 10 022 465

## Beschreibung

Die Erfindung betrifft ein Verfahren zur Auftrennung eines Roh-C₄-Schnittes durch Extraktivdestillation mit einem selektiven Lösungsmittel.

Der Begriff C₄-Schnitt bezeichnet Gemische von Kohlenwasserstoffen mit überwiegend 4 Kohlenstoffatomen pro Molekül. C₄-Schnitte werden beispielsweise bei der Herstellung von Ethylen und/oder Propylen durch thermisches Spalten, üblicherweise in Steamcrackern, insbesondere Naphtha-Crackern oder FCC-Crackern (Fluidized Catalytic Cracking) einer Petroleumfraktion, wie verflüssigtes Petroleumgas, Leichtbenzin oder Gasöl, erhalten. Weiterhin werden C₄-Schnitte bei der katalytischen Dehydrierung von n-Butan und/oder n-Buten erhalten. C₄-Schnitte enthalten in der Regel Butane, n-Buten, Isobuten, 1,3-Butadien, daneben geringe Mengen an sonstigen Kohlenwasserstoffen, darunter 1,2-Butadien, C₅-Kohlenwasserstoffe sowie C₄-Acetylene (Butine) insbesondere 1-Butin (Ethylacetylen) und Butenin (Vinylacetylen). Dabei beträgt der 1,3-Butadiengehalt von C₄-Schnitten aus Steamcrackern im allgemeinen 20 bis 70 Gew.-%, insbesondere 35 bis 65 Gew.-%, während der Gehalt an C₄-Acetylenen (Vinylacetylen und Ethylacetylen) im allgemeinen 5 Gew.-% nicht übersteigt.

Die Auftrennung von C₄-Schnitten ist wegen der geringen Unterschiede in den relativen Flüchtigkeiten der Komponenten ein kompliziertes destillationstechnisches Problem. Daher wird die Auftrennung durch eine so genannte Extraktivdestillation durchgeführt, d. h. eine Destillation unter Zugabe eines selektiven Lösungsmittels (auch als Extraktionsmittel bezeichnet), das einen höheren Siedepunkt als das aufzutrennende Gemisch aufweist und das die Unterschiede in den relativen Flüchtigkeiten der aufzutrennenden Komponenten erhöht.

Es sind eine Vielzahl von Verfahren zur Auftrennung von C₄-Schnitten mittels Extraktivdestillation unter Verwendung von selektiven Lösungsmitteln bekannt. Ihnen ist gemeinsam, dass sich durch Gegenstromführung des aufzutrennenden C₄-Schnittes in Dampfform mit dem flüssigen selektiven Lösungsmittel bei geeigneten thermodynamischen Bedingungen, in der Regel bei niedrigen Temperaturen, häufig im Bereich von 20 bis 80°C und bei moderaten Drücken, häufig bei Normaldruck bis 6 bar, das selektive Lösungsmittel mit den Komponenten aus dem C₄-Schnitt belädt, zu denen es eine höhere Affinität hat, wogegen die Komponenten, zu denen das selektive Lösungsmittel eine geringere Affinität hat, in der Dampfphase verbleiben und als Kopfstrom abgezogen werden. Aus dem beladenen Lösungsmittelstrom werden anschließend unter geeigneten thermodynamischen Bedingungen, d.h. bei höherer Temperatur und/oder niedrigerem Druck gegenüber dem ersten Verfahrensschritt, in einem oder mehreren weiteren Verfahrensschritten die Komponenten fraktioniert aus dem selektiven Lösungsmittel freigesetzt.
In den Verfahren zur thermischen Auftrennung von C₄-Schnitten sind insbesondere dessen Komponenten C₄-Acetylene problematisch, da sie einer der Hauptverursacher für das Apparate-Fowling sind sowie in weiten Konzentrationsbereichen selbstzersetzungsgefährdet sind.

Es wurden daher Verfahren zur Auftrennung von C₄-Schnitten entwickelt, wonach die C₄-Acetylene in einem ersten Verfahrensschritt, durch so genannte Front-end-Hydrierung, umgesetzt werden. Die Front-end-Hydrierung hat darüber hinaus den weiteren Vorteil, dass durch die Hydrierung der C₄-Acetylene zusätzliches Wertprodukt 1,3-Butadien gewonnen wird.

Ein derartiges Verfahren ist in Proc.-Ethylene Prod. Conf. 5 (1996), Seiten 631 bis 636 beschrieben. Danach wird unter Verwendung eines sogenannten KLP-Katalysators, das heißt einem Katalysator, der feinverteilte Kupfer-Partikel auf einem hochreinen γ-Aluminiumoxid als Träger mit definierter Porenstruktur enthält, ein hoher Vinylacetylenumsatz bei niedrigem Butadienverlust erreicht, bei hohen Katalysatorstandzeiten. Durch die vorgeschaltete Selektivhydrierung kann die zweistufige Butadien-Extraktivdestillation auf einen Einstufenprozeß vereinfacht sowie der apparative Aufwand in der nachgeschalteten Reindestillation um eine Trennkolonne reduziert werden. Das Verfahren hat jedoch den Nachteil, daß eine separate Anlage zur vorgeschalteten Selektivhydrierung der acetylenischen Verunreinigungen erforderlich ist.

Aus der US 4,277,313 ist ein weiteres Verfahren zur Gewinnung von 1,3-Butadien bekannt, wonach zunächst eine Selektivhydrierung und anschließend eine Extraktivdestillation des 1,3-Butadiens durchgeführt werden. Die Selektivhydrierung kann in Flüssig- oder Gasphase, in Gegenwart von Katalysatoren der VIII. Gruppe des Periodensystems, beispielsweise an einem Palladium/Aluminiumoxid-Katalysator durchgeführt werden. Als Extraktionsmittel werden Dimethyl- oder Diethylformamid, N-Methylpyrrolidon, Furfurol oder Acetonitril genannt. Das Verfahren weist, analog zum vorstehend beschriebenen, den Nachteil auf, daß für die vorgeschaltete Selektivhydrierung eine getrennte Anlage erforderlich ist.

Aus US 6,040,489 ist ein Verfahren zur Abtrennung von 1,3-Butadien aus einem C₄-Schnitt bekannt, wobei der C₄-Schnitt in einer Kolonne hydriert und mit einem Lösungsmittel selektiv extrahiert wird, aus der Kolonne ein mindestens die Butane und Butene umfassender Strom als Kopfstrom abgezogen und das mit Butadienen beladene Lösungsmittel über Sumpf abgezogen und anschließend in einer Lösungsmittel-Strippkolonne in einen butadienhaltigen Kopfstrom und einen lösungsmittelhaltigen Sumpfstrom aufgetrennt wird.
Der butadienhaltige Kopfstrom wird in einer Butadien-Destillationskolonne in einen 1,3-butadienhaltigen Kopfstrom und einen 1,2-butadienhaltigen Sumpfstrom aufgetrennt.

Nach dem Verfahren der DE-A 100 22 465.2 wird ein C₄-Schnitt in einer Trennwandkolonne oder in thermisch gekoppelten Kolonnen einer Extraktivdestillation sowie einer Selektivhydrierung an einem heterogenen Katalysator, unter Erhalt eines Roh-1,3-Butadienstromes, unterworfen.

Die bekannten Front-end-Verfahren zur Entfernung der C₄-Acetylene aus C₄-Schnitten durch Selektivhydrierung an heterogenen Katalysatoren haben den Nachteil, dass die nicht unerheblichen Katalysatorkosten aufgewendet werden müssen, wobei die bekannten Katalysatoren darüber hinaus häufig keine hohen Standzeiten aufweisen. Besonders kritisch ist, dass bei Ausfall des Katalysators die gesamte Anlage zur thermischen Trennung des C₄-Schnittes ausfällt.

Es war daher Aufgabe der Erfindung, ein Verfahren zur Front-end-Abtrennung der C₄-Acetylene aus C₄-Schnitten zur Verfügung zu stellen, das die oben genannten Nachteile nicht aufweist.

Die Aufgabe wird durch ein Verfahren zur Auftrennung eines Roh-C₄-Schnittes, enthaltend Butane, Butene, 1,3-Butadien sowie geringe Mengen an sonstigen Kohlenwasserstoffen, darunter C₄-Actylene, 1,2-Butadien und C₅-Kohlenwaserstoffe, durch Extraktivdestillation mit einem selektiven Lösungsmittel gelöst, wonach einer ersten Extraktivdestillationskolonne der Roh-C₄-Schnitt im mittleren Bereich derselben und das selektive Lösungsmittel oberhalb des Roh-C₄-Schnittes zugeführt wird. Aus der ersten Extraktivdestillationskolonne wird unterhalb der Zuführung des Roh-C₄-Schnittes ein dampfförmiger Seitenstrom abgezogen der die C₄-Acetylene und daneben 1,3-Butadien, 1,2-Butadien, C₅-Kohlenwasserstoffe und selektives Lösungsmittel enthält, wobei die Konzentration der C₄-Acetylene im dampfförmigen Seitenstrom unterhalb der Selbstzersetzungsgrenze derselben liegt, sowie ein Kopfstrom, der die im selektiven Lösungsmittel weniger als die C₄-Acetylene löslichen Komponenten des C₄-Schnittes enthält.

Es wurde gefunden, dass es wirtschaftlich sinnvoll und verfahrenstechnisch möglich ist, in einer Extraktivdestillationskolonne geeignete Betriebsbedingungen, insbesondere bezüglich der Art des selektiven Lösungsmittels, der Menge desselben, von Temperatur, Druck und Anzahl der theoretischen Trennstufen so einzustellen, dass man die C₄-Acetylene, das heißt die Komponenten aus dem C₄-Schnitt, zu denen das selektive Lösungsmittel die höchste Affinität hat, selektiv abtrennen kann. Dabei handelt es sich um eine für Extraktivdestillationen ungewöhnliche Verfahrensführung.

Ein typischer Roh-C₄-Schnitt aus einem Naphtha-Cracker weist die folgende Zusammensetzung in Gewichtsprozenten auf:

| | |
|---|---|
| Propan | 0 - 0,5 |
| Propen | 0 - 0,5 |
| Propadien | 0 - 0,5 |
| Propin | 0 - 0,5 |
| n-Butan | 3 - 10 |
| i-Butan | 1 - 3 |
| 1-Buten | 10 - 20 |
| i-Buten | 10 - 30 |
| trans-2-Buten | 2 - 8 |
| cis-2-Buten | 2 - 6 |
| 1,3-Butadien | 35 - 65 |
| 1,2-Butadien | 0,1 - 1 |
| Ethylacetylen | 0,1 - 2 |
| Vinylacetylen | 0,1 - 3 |
| C5 | 0 - 0,5 |

Roh-C₄-Schnitte aus Naphtha-Crackern enthalten somit überwiegend Butane, Butene und 1,3-Butadien. Darüber hinaus sind geringe Mengen an sonstigen Kohlenwasserstoffen enthalten. C₄-Acetylene sind häufig bis zu einem Anteil von 5 Gew.-%, oder auch bis zu 2 Gew.-% enthalten.

Für die eingangs bereits definierte Extraktivdestillation kommen als selektive Lösungsmittel generell Substanzen oder Gemische in Frage, die einen höheren Siedepunkt als das aufzutrennende Gemisch sowie eine größere Affinität zu konjugierten Doppelbindungen und Dreifachbindungen als zu einfachen Doppelbindungen sowie Einfachbindungen aufweisen, bevorzugt dipolare, besonders bevorzugt dipolar-aprotische Lösungsmittel. Aus apparatetechnischen Gründen werden wenig oder nicht korrosive Substanzen bevorzugt.

Geeignete selektive Lösungsmittel für das erfindungsgemäße Verfahren sind zum Beispiel Butyrolacton, Nitrile wie Acetonitril, Propionitril, Methoxypropionitril, Ketone wie Aceton, Furfurol, N-alkylsubstituierte niedere aliphatische Säureamide, wie Dimethylformamid, Diethylformamid, Dimethylacetamid, Diethylacetamid, N-Formylmorpholin, N-alkylsubstituierte cyclische Säureamide (Lactame) wie N-Alkylpyrrolidone, insbesondere N-Methylpyrrolidon. Im Allgemeinen werden N-alkylsubstituierte niedere aliphatische Säureamide oder N-alkylsubstituierte cyclische Säureamide verwendet. Besonders vorteilhaft sind Dimethylformamid, Acetonitril, Furfurol und insbesondere N-Methylpyrrolidon.

Es können jedoch auch Mischungen dieser Lösungsmittel untereinander, zum Beispiel von N-Methylpyrrolidon mit Acetonitril, Mischungen dieser Lösungsmittel mit Colösungsmitteln wie Wasser und/oder tert.-Butylether, zum Beispiel Methyl-tert.-butylether, Ethyl-tert.-butylether, Propyl-tert.-butylether, n- oder iso-Butyl-tert.-butylether eingesetzt werden.

Besonders geeignet ist N-Methylpyrrolidon, bevorzugt in wässriger Lösung, insbesondere mit 8 bis 10 Gew.-% Wasser, besonders bevorzugt mit 8,3 Gew.-% Wasser.

Bezüglich der zur Durchführung der Extraktivdestillation einsetzbaren Kolonnen gibt es grundsätzlich keine Einschränkungen.

Der Kolonne wird in ihrem mittleren Bereich der C₄-Schnitt und das selektive Lösungsmittel oberhalb der Zuführung des C₄-Schnittes zugeführt.

Die Kolonne ist mit trennwirksamen Einbauten bestückt. Hierfür können alle bekannten Arten eingesetzt werden. Oberhalb der Zuführung des selektiven Lösungsmittels sind bevorzugt ein oder mehrere Böden angeordnet.

Erfindungsgemäß wird aus der ersten Extraktivdestillationskolonne ein Seitenstrom dampfförmig abgezogen, der die C₄-Acetylene und daneben 1,3 Butadien, 1,2-Butadien, C₅-Kohlenwasserstoffe und selektives Lösungsmittel enthält, wobei die Kolonne in der Weise betrieben werden muss, dass die Konzentration der C₄-Acetylene im dampfförmigen Seitenstrom unterhalb der Selbstzersetzungsgrenze derselben liegt. Hierfür ist in der Regel eine Verdünnung auf unterhalb von 30 Mol-% C₄-Acetylenen ausreichend.

Der dampfförmige Seitenstrom enthält selektives Lösungsmittel in einem Anteil, der dem thermodynamischen Gleichgewicht entspricht. Die Seitenkolonne wird als reine Verstärkungskolonne betrieben und dient der Rückgewinnung von selektivem Lösungsmittel. Sie muss so betrieben werden, dass an jedem Punkt der Kolonne eine ausreichende Verdünnung der Acetylene unterhalb des selbstzersetzungsgefährdeten Konzentrationsbereiches gewährleistet ist.

Aus der ersten Extraktivdestillationskolonne wird ein Kopfstrom abgezogen, der die im selektiven Lösungsmittel weniger als die C₄-Acetylene löslichen Komponenten aus dem C4-Schnitt enthält. Dieser Strom wird bevorzugt in einem Kondensator am Kolonnenkopf kondensiert, teilweise als Rücklauf wieder auf die Kolonne aufgegeben und im Übrigen bevorzugt in einer zweiten Extraktivdestillationskolonne weiter aufgearbeitet. Energetisch besonders vorteilhaft ist eine Teilkondensation, wobei der kondensierte Anteil als Rücklauf auf die Kolonne dient und der dampfförmige Anteil einer zweiten Extraktivdestillationskolonne als Feedstrom zugeführt wird, in der man die Auftrennung in Raffinat 1 und Roh-1,3-Butadien durchführt.

Der Sumpfstrom aus der ersten Extraktivdestillationskolonne, der überwiegend das selektive Lösungsmittel enthält, wird bevorzugt zur Wärmeintegration in die erste Extraktivdestillationskolonne genutzt, kondensiert und in die erste Extraktivdestillationskolonne recycliert.

Zur Wärmeintegration ist es möglich, den heißen Sumpfstrom aus der ersten Extraktivdestillationskolonne mit dem Roh-C₄-Schnitt abzukühlen. Zusätzlich oder alternativ ist es möglich, dass man aus der ersten Extraktivdestillationskolonne von einer Trennstufe, die eine oder mehrere Trennstufen unterhalb des dampfförmigen Seitenabzugs liegt, Flüssigkeit oder einen Teilstrom der Flüssigkeit abzieht, durch indirekten Wärmetausch mit dem Sumpfstrom aus der ersten Extraktivdestillationskolonne erwärmt und/oder verdampft und auf der selben Trennstufe oder oberhalb derselben in die erste Extraktivdestillationskolonne zurückführt, wobei die Trennstufe, von der die Flüssigkeit oder der Flüssigkeitsteilstrom abgezogen wird, dergestalt gewählt wird, dass der Energiebedarf für die erste Extraktivdestillationskolonne minimal ist.
Hierbei bezeichnet der Begriff Raffinat 1 in bekannter Weise einen Butane und Butene enthaltenden Strom.

Als Roh-1,3-Butadien wird ein Kohlenwasserstoffgemisch bezeichnet, das das Wertprodukt 1,3-Butadien in einem Anteil von mindestens 90 Gew.-%, bevorzugt mindestens 95 Gew.-%, besonders bevorzugt mindestens 98 Gew.-%, Rest Verunreinigungen, enthält.

Bezüglich der trennwirksamen Einbauten gelten für die zweite Extraktivdestillationskolonne die Ausführungen zur ersten Extraktivdestillationskolonne analog.

Aus der zweiten Extraktivdestillationskolonne zieht man bevorzugt einen Kopfstrom oder einen Strom in der Nähe des Kopfes der Kolonne ab, kondensiert denselben in einem Kondensator, gibt das Kondensat teilweise als Rücklauf wieder auf die zweite Extraktivdestillationskolonne auf und zieht es im übrigen als Raffinat 1 ab.

Aus der zweiten Extraktivdestillationskolonne zieht man einen Seitenstrom ab, aus dem man bevorzugt, durch Zuführung desselben in eine zweite kurze Seitenkolonne, selektives Lösungsmittel abtrennt, das man in die zweite Extraktivdestillationskolonne recycliert und aus der man einen Kopfstrom gewinnt, den man in einem Kondensator am Kolonnenkopf kondensiert, teilweise als Rücklauf wieder auf die Kolonne aufgibt und im übrigen als Roh-1,3-Butadien abzieht.

In der zweiten Extraktivdestillationskolonne ist eine Wärmeintegration analog zur ersten Extraktivdestillationskolonne möglich, indem man aus der zweiten Extraktivdestillationskolonne von einer Trennstufe, die eine oder mehrere Trennstufen unterhalb des Seitenabzugs liegt, Flüssigkeit oder einen Teilstrom der Flüssigkeit abzieht, durch indirekten Wärmetausch mit dem Sumpfstrom aus der zweiten Extraktivdestillationskolonne erwärmt und/oder verdampft und auf derselben Trennstufe oder oberhalb derselben in die zweite Extraktivdestillationskolonne zurückführt, wobei die Trennstufe, von der die Flüssigkeit oder der Flüssigkeitsteilstrom abgezogen wird, dergestalt gewählt wird, dass der Energiebedarf für die zweite Extraktivdestillationskolonne minimal ist.

Die Erfindung wird im Folgenden anhand einer Zeichnung und eines Ausführungsbeispiels näher erläutert.

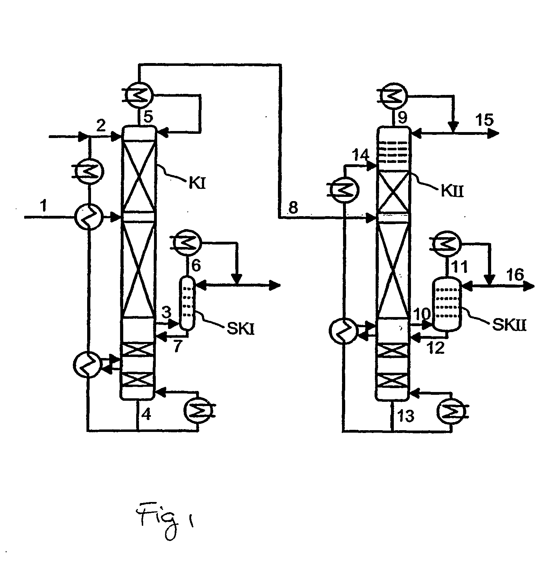

Die einzige Figur zeigt die schematische Darstellung einer Anlage nach der Erfindung zur Auftrennung eines C₄-Schnittes durch Extraktivdestillation.

Einer ersten Extraktivdestillationskolonne K I wird ein Roh-C₄-Schnitt, Strom 1, im mittleren Bereich derselben und selektives Lösungsmittel, Strom 2, oberhalb des Roh-C₄-Schnittes zugeführt. Über einen dampfförmigen Seitenabzug wird unterhalb der Zuführung des Stromes 1 ein C₄-Acetylene enthaltender Strom 3 abgezogen, der einer ersten Seitenkolonne SK I zugeführt wird. In der Seitenkolonne SK I wird destillativ ein die Acetylene enthaltender Kopfstrom 6 abgetrennt sowie ein Sumpfstrom 7, der selektives Lösungsmittel enthält und der in die erste Extraktivdestillationskolonne K I recycliert wird.

Der Sumpfstrom 4 aus der ersten Extraktivdestillationskolonne K I, der überwiegend selektives Lösungsmittel enthält, wird zur Wärmeintegration mit einem Flüssigkeitsstrom, der aus dem unteren Bereich der ersten Extraktivdestillationskolonne K I abgezogen wird, sowie zur Vorerwärmung des Roh-C₄-Schnittes, Strom 1, genutzt, kondensiert und abgekühlt und erneut der ersten Extraktivdestillationskolonne K I zugeführt.

Der Kopfstrom 5 aus der ersten Extraktivdestillationskolonne wird in einem Kondensator am Kolonnenkopf teilkondensiert, das Kondensat als Rücklauf wieder auf die Kolonne aufgegeben und der dampfförmige Anteil im Übrigen als Strom 8 abgezogen.

In der zweiten Extraktivdestillationskolonne K II wird der Strom 8 durch Gegenstromführung mit dem selektiven Lösungsmittel, Strom 14, destillativ in einen Kopfstrom 9 aufgetrennt, der kondensiert, teilweise als Rücklauf wieder auf die Kolonne K II aufgegeben und im übrigen als Raffinat 1, Strom 15 abgezogen wird sowie in einen Seitenstrom 10, aus dem nach Abtrennung von Lösungsmittel in einer zweiten kurzen Seitenkolonne SK II Roh-1,3-Butadien gewonnen wird. In der zweiten Seitenkolonne SK II wird ein Kopfstrom 11 abgezogen, in einem Kondensator am Kolonnenkopf kondensiert, teilweise als Rücklauf wieder auf die Kolonne aufgegeben und im übrigen als Roh-1,3-Butadien, Strom 16, abgezogen sowie ein Sumpfstrom 12, der überwiegend das selektive Lösungsmittel enthält und der in die zweite Extraktivdestillationskolonne K II recycliert wird. Aus der zweiten Extraktivdestillationskolonne K II wird ein Sumpfstrom 13 abgezogen, zur Wärmeintegration mit einem Flüssigkeitsstrom aus dem unteren Bereich der zweiten Extraktivdestillationskolonne K II genutzt, kondensiert und anschließend erneut als Strom 14 auf die zweite Extraktivdestillationskolonne aufgegeben.

### Ausführungsbeispiel

Einer Extraktivdestillationskolonne K I mit 28 theoretischen Trennstufen, mit Zählung der Trennstufen von unten, wird auf der 15ten theoretischen Trennstufe ein Roh-C₄-Strom, Bezugsziffer 1 in der Figur, in einem Mengenstrom von 32 t/h mit folgenden Komponenten mit Gewichtsanteilen > als jeweils 0,01 Gew.-% zugeführt:

| | |
|---|---|
| n-Butan | 5,75 |
| i-Butan | 2,45 |
| 1-Buten | 13,89 |
| i-Buten | 25,65 |
| trans-2-Buten | 4,44 |
| cis-2-Buten | 2,96 |
| 1,3-Butadien | 43,84 |
| 1,2-Butadien | 0,14 |
| Ethylacetylen | 0,13 und |
| Vinylacetylen | 0,74. |

Die Kolonne wird bei einem Kopfdruck von 4,5 bar absolut und einer Temperatur am Kolonnenkopf von 58,8 °C betrieben.

Auf der obersten Stufe der Extraktivdestillationskolonne K I wird ein Mengenstrom von 120 t/h des Extraktivlösungsmittels N-Methylpyrrolidon (NMP), mit 8,3 Gew.-% Wasser Strom 2, zugeführt.

Von der dritten theoretischen Trennstufe wird ein Mengenstrom von 224 kg/h, Bezugsziffer 3 in der Figur, mit folgenden Komponenten mit Gewichtsanteilen jeweils > 0,01 Gew.-% abgezogen:

| | |
|---|---|
| 1,3-Butadien | 2,45 |
| 1,2-Butadien | 1,21 |
| Ethylacetylen | 1,67 |
| Vinylacetylen | 10,40 |
| Wasser | 70,10 und |
| NMP | 14,08. |

Aus diesem Strom wird in der Seitenkolonne SK I, die als reine Verstärkungskolonne betrieben wird, mit dem Rücklauf das Lösungsmittel ausgewaschen. Dabei wird ein Kopfstrom, Bezugsziffer 6 in der Figur, erhalten, mit folgenden Komponenten in Gewichtsanteilen von jeweils > 0,01 Gew.-%:

| | |
|---|---|
| 1,3-Butadien | 3,24 |
| 1,2-Butadien | 1,60 |
| Ethylacetylen | 2,20 |
| Vinylacetylen | 13,56 |
| C₅-Kohlenwasserstoffe | 0,16 und |
| Wasser | 79,24. |

Die teuer zugekaufte Komponente N-Methylpyrrolidon des Extraktivlösungsmittels wird bis auf 1 ppm im Kopfstrom der Seitenkolonne vollständig abgetrennt und der ersten Extraktivdestillationskolonne K I erneut zugeführt.

Über den Kopfstrom der Seitenkolonne SK I können somit die Acetylene (Ethylacetylen, Vinylacetylen) praktisch ohne Verluste an der teuren Komponente N-Methylpyrrolidon aus dem Verfahren ausgeschleust werden.

## Patentansprüche

1. Verfahren zur Auftrennung eines Roh-C₄-Schnittes, enthaltend Butane, Butene, 1,3-Butadien sowie geringe Mengen an sonstigen Kohlenwasserstoffen, darunter C₄-Acetylene, 1,2-Butadien und C₅-Kohlenwasserstoffe durch Extraktivdestillation mit einem selektiven Lösungsmittel, wobei einer ersten Extraktivdestillationskolonne (K I) der Roh-C₄-Schnitt (1) im mittleren Bereich derselben und das selektive Lösungsmittel (2) oberhalb des Roh-C₄-Schnittes (1) zugeführt wird und aus der ersten Extraktivdestillationskolonne (K I) unterhalb der Zuführung des Roh-C₄-Schnittes (1) ein dampfförmiger Seitenstrom (3) abgezogen wird, der die C₄-Acetylene und daneben 1,3-Butadien, 1,2-Butadien, C₅-Kohlenwasserstoffe und selektives Lösungsmittel enthält, wobei die Konzentration der C₄-Acetylene im dampfförmigen Seitenstrom (3) unterhalb der Selbstzersetzungsgrenze derselben liegt, sowie ein Kopfstrom (5), der die im selektiven Lösungsmittel weniger als die C₄-Acetylene löslichen Komponenten des Roh-C₄-Schnittes enthält.

2. Verfahren nach Anspruch 1, **dadurch gekennzeichnet, dass** der dampfförmige Seitenstrom (3) einer ersten Seitenkolonne (SK I) zugeführt wird, aus der ein Kopfstrom (6), enthaltend die C₄-Acetylene abgezogen wird, der in einem Kondensator am Kopf der ersten Seitenkolonne (SK I) kondensiert, teilweise als Rücklauf wieder auf die erste Seitenkolonne (SK I) aufgegeben und im übrigen abgezogen wird sowie ein Sumpfstrom (7), enthaltend das selektive Lösungsmittel, der der ersten Extraktivdestillationskolonne (K I) erneut zugeführt wird.

3. Verfahren nach Anspruch 1 oder 2, **dadurch gekennzeichnet, dass** aus der ersten Extraktivdestillationskolonne (K I) ein Sumpfstrom (4) abgezogen wird, der durch indirekten Wärmetausch mit dem Roh-C₄-Schnitt (1) abgekühlt, in einem Kondensator kondensiert und erneut als Strom (2) auf die erste Extraktivdestillationskolonne (K I) aufgegeben wird.

4. Verfahren nach einem der Ansprüche 1 bis 3, **dadurch gekennzeichnet, dass** man aus der ersten Extraktivdestillationskolonne (K I) von einer Trennstufe, die eine oder mehrere Trennstufen unterhalb des dampfförmigen Seitenabzugs (3) liegt, Flüssigkeit oder einen Teilstrom der Flüssigkeit abzieht, durch indirekten Wärmetausch mit dem Sumpfstrom (4) aus der ersten Extraktivdestillationskolonne (K I) erwärmt und/oder verdampft und auf der selben Trennstufe oder oberhalb derselben in die erste Extraktivdestillationskolonne (K I) zurückführt, wobei die Trennstufe, von der die Flüssigkeit oder der Flüssigkeitsteilstrom abgezogen wird, dergestalt gewählt wird, dass der Energiebedarf für die erste Extraktivdestillationskolonne (K I) minimal ist.

5. Verfahren nach einem der Ansprüche 1 bis 4, **dadurch gekennzeichnet, dass** man den Kopfstrom (5) aus der ersten Extraktivdestillationskolonne (K I) in einem Kondensator am Kopf der ersten Extraktivdestillationskolonne (K I) kondensiert, teilweise als Rücklauf zurückführt und den übrigen Teil des kondensierten Kopfstromes (8) einer zweiten Extraktivdestillationskolonne (K II) zuführt, in der man die Auftrennung in Raffinat 1 und Roh-1,3-Butadien durchführt.

6. Verfahren nach Anspruch 5, **dadurch gekennzeichnet, dass** man im Kondensator am Kopf der ersten Extraktivdestillationskolonne (K I) eine Teilkondensation durchführt, den kondensierten Anteil aus dem Kopfstrom (5) der ersten Extraktivdestillationskolonne (K I) als Rücklauf verwendet und den dampfförmigen Anteil derselben der zweiten Extraktivdestillationskolonne (K II) zuführt.

7. Verfahren nach Anspruch 5 oder 6, **dadurch gekennzeichnet, dass** man aus der zweiten Extraktivdestillationskolonne (K II) einen Kopfstrom (9) abzieht, in einem Kondensator kondensiert, teilweise als Rücklauf wieder auf die zweite Extraktivdestillationskolonne (K II) gibt und im übrigen als Raffinat 1 abzieht und dass man aus der zweiten Extraktivdestillationskolonne (K II), unterhalb der Zuführung des Stromes (8) einen Seitenstrom (10) abzieht, den man bevorzugt einer zweiten Seitenkolonne (SK II) zuführt, aus der man einen Kopfstrom abzieht, kondensiert, teilweise als Rücklauf wieder auf die zweite Seitenkolonne (SK II) aufgibt, im übrigen als Roh-1,3-Butadien-Strom abzieht sowie einen Stumpfstrom (12) umfassend das selektive Lösungsmittel, den man erneut der zweiten Extraktivdestillationskolonne (K II) zuführt.

8. Verfahren nach einem der Ansprüche 5 bis 7, **dadurch gekennzeichnet, dass** man aus der zweiten Extraktivdestillationskolonne (K II) von einer Trennstufe, die eine oder mehrere Trennstufen unterhalb des Seitenabzugs (10) liegt, Flüssigkeit oder einen Teilstrom der Flüssigkeit abzieht, durch indirekten Wärmetausch mit dem Sumpfstrom (13) aus der zweiten Extraktivdestillationskolonne (K II) erwärmt und/oder verdampft und auf derselben Trennstufe oder oberhalb derselben in die zweite Extraktivdestillationskolonne (K II) zurückführt, wobei die Trennstufe, von der die Flüssigkeit oder der Flüssigkeitsteilstrom abgezogen wird, dergestalt gewählt wird, dass der Energiebedarf für die zweite Extraktivdestillationkolonne (K II) minimal ist.

## Claims

1. A process for fractionating a crude C₄ fraction comprising butanes, butenes, 1,3-butadiene and small amounts of other hydrocarbons including C₄-acetylenes, 1,2-butadiene and C₅-hydrocarbons by extractive distillation using a selective solvent, wherein the crude C₄ fraction (1) is fed into the middle region of a first extractive distillation column (K I) and the selective solvent (2) is fed into the column at a point above that at which the crude C₄ fraction (1) is introduced and a gaseous side stream (3) which comprises the C₄-acetylenes together with 1,3-butadiene, 1,2-butadiene, C₅-hydrocarbons and selective solvent and in which the concentration of the C₄-acetylenes is below the spontaneous decomposition limit is taken off from the first extractive distillation column (K I) at a point below the feed point for the crude C₄ fraction (1) and an overhead stream (5) comprising the components of the crude C₄ fraction which are less soluble than the C₄-acetylenes in the selective solvent is taken off from the top of the first extractive distillation column.

2. The process according to claim 1, wherein the gaseous side stream (3) is fed to a first side column (SK I) in which it is separated into an overhead stream (6) which comprises the C₄-acetylenes and is condensed in a condenser at the top of the first side column (SK I) and part of it is returned as runback to the first side column (SK I) while the remainder is taken off and a bottom stream (7) which comprises the selective solvent and is returned to the first extractive distillation column (K I).

3. The process according to claim 1 or 2, wherein a bottom stream (4) is taken off from the first extractive distillation column (K I) and is cooled by indirect heat exchange with the crude C₄ fraction (1), condensed in a condenser and returned as stream (2) to the first extractive distillation column (K I).

4. The process according to any of claims 1 to 3, wherein liquid or a substream of the liquid is taken off from the first extractive distillation column (K I) at a theoretical plate which is one or more theoretical plates below the point at which the gaseous side stream (3) is taken off, the liquid is heated and/or vaporized by indirect heat exchange with the bottom stream (4) from the first extractive distillation column (K I) and is returned to the first extractive distillation column (K I) on the same theoretical plate or above this point, with the theoretical plate from which the liquid or liquid substream is taken off being chosen so that the energy requirement for the first extractive distillation column (K I) is minimized.

5. The process according to any of claims 1 to 4, wherein the overhead stream (5) from the first extractive distillation column (K I) is condensed in a condenser at the top of the first extractive distillation column (K I) and part of it is returned as runback while the remainder of the condensed overhead stream (8) is fed to a second extractive distillation column (K II) in which it is separated into raffinate 1 and crude 1,3-butadiene.

6. The process according to claim 5, wherein a partial condensation is carried out in the condenser at the top of the first extractive distillation column (K I) and the condensed portion of the overhead stream (5) from the first extractive distillation column (K I) is used as runback while the gaseous portion of it is fed to the second extractive distillation column (K II).

7. The process according to claim 5 or 6, wherein an overhead stream (9) is taken off from the second extractive distillation column (K II), condensed in a condenser and part of it is returned as runback to the second extractive distillation column (K II) while the remainder is taken off as raffinate 1, and a side stream (10) is taken off from the second extractive distillation column (K II) below the feed point for the stream (8) and this is preferably fed to a second side column (SK II) in which it is separated into an overhead stream which is condensed and part of it is returned as runback to the second side column (SK II) while the remainder is taken off as crude 1,3-butadiene stream and a bottom stream (12) which comprises the selective solvent and is returned to the second extractive distillation column (K II).

8. The process according to any of claims 5 to 7, wherein liquid or a substream of the liquid is taken off from the second extractive distillation column (K II) at a theoretical plate which is one or more theoretical plates below the side offtake (10), the liquid is heated and/or vaporized by indirect heat exchange with the bottom stream (13) from the second extractive distillation column (K II) and is returned to the second extractive distillation column (K II) on the same theoretical plate or above this point, with the theoretical plate from which the liquid or liquid substream is taken off being chosen so that the energy requirement for the second extractive distillation column (K II) is minimized.

## Revendications

1. Procédé pour le fractionnement d'une coupe C₄ brute comprenant des butanes, des butènes, du 1,3-butadiène et de petites quantités d'autres hydrocarbures incluant des acétylènes C₄, du 1,2-butadiène et des hydrocarbures C₅ par distillation extractive en utilisant un solvant sélectif, dans lequel la coupe C₄ brute (1) est amenée dans la zone centrale d'une première colonne de distillation extractive (K I) et le solvant sélectif (2) est introduit au-dessus de la coupe C₄ brute (1) et un courant latéral sous forme vapeur (3) qui comprend les acétylènes C₄ associés au 1,3-butadiène, au 1,2-butadiène, aux hydrocarbures C₅ et au solvant sélectif est prélevé de la première colonne de distillation extractive (K I) à un point en dessous du point d'amenée de la coupe C₄ brute (1), où la concentration des acétylènes C₄ dans le courant latéral sous forme vapeur (3) est en dessous de la limite de décomposition spontanée et un courant de tête (5) comprenant les composants de la coupe C₄ brute qui sont moins solubles que les acétylènes C₄ dans le solvant sélectif.

2. Procédé selon la revendication 1, **caractérisé en ce que** le courant latéral sous forme vapeur (3) est amené dans une première colonne latérale (SK I) de laquelle est prélevé un courant de tête (6) qui comprend des acétylènes C₄ et est condensé dans un condenseur en tête de la première colonne latérale (SK I) et une partie de celui-ci est retournée comme reflux à la première colonne latérale (SK I) et le restant est prélevé, et un courant de queue (7) qui comprend le solvant sélectif et est retourné à la première colonne de distillation extractive (K I).

3. Procédé selon la revendication 1 ou 2, **caractérisé en ce qu'**un courant de queue (4) est prélevé de la première colonne de distillation extractive (K I) et est refroidi par échange thermique indirect avec la coupe C₄ brute (1), condensé dans un condenseur et retourné comme courant (2) à la première colonne de distillation extractive (K I).

4. Procédé selon l'une quelconque des revendications 1 à 3, **caractérisé en ce que** le liquide ou un courant partiel du liquide est prélevé de la première colonne de distillation extractive (K I) à partir d'un plateau théorique qui est situé un ou plusieurs plateaux théoriques en dessous du prélèvement latéral sous forme vapeur, chauffé et/ou vaporisé par échange thermique indirect avec le courant de fond (4) provenant de la première colonne de distillation extractive (K I) et est retourné à la première colonne de distillation extractive (K I) sur le même plateau théorique ou au-dessus de celui-ci, le plateau théorique à partir duquel le liquide ou le courant partiel de liquide est prélevé étant choisi de sorte que l'exigence d'énergie pour la première colonne de distillation extractive (K I) soit minimale.

5. Procédé selon l'une quelconque des revendications 1 à 4, **caractérisé en ce que** le courant de tête (5) provenant de la première colonne de distillation extractive (K I) est condensé dans un condenseur en tête de la première colonne de distillation extractive (K I) et une partie de celui-ci est retourné comme reflux et le restant du courant de tête condensé (8) est amené à une seconde colonne de distillation extractive (K II) dans laquelle l'on procède à la séparation en un raffinat 1 et 1,3-butadiène en brut.

6. Procédé selon la revendication 5, **caractérisé en ce qu'**une condensation partielle est effectuée dans le condenseur en tête de la première colonne de distillation extractive (K I) et la partie condensée de le courant de tête (5) de la première colonne de distillation extractive (K I) est utilisée comme reflux et la partie sous forme vapeur de celui-ci est amenée à la seconde colonne de distillation extractive (K II).

7. Procédé selon la revendication 5 ou 6, **caractérisé en ce qu'**une courant de tête (9) est prélevé de la seconde colonne de distillation extractive (K II), condensé dans un condenseur et une partie de celui-ci est retournée comme reflux à la seconde colonne de distillation extractive (K II) et le restant est prélevé comme raffinat 1, et **en ce que** l'on prélève une courant latéral (10) de la seconde colonne de distillation extractive (II) en dessous du point d'amenée du courant (8) qui est, de préférence, amené à une seconde colonne latérale (SK II) à partir de laquelle un courant de tête est prélevé, est condensé et une partie de celui-ci est retournée comme reflux à la seconde colonne latérale (SK II) et le restant est prélevé comme courant de 1,3-butadiène brut et que l'on prélève également un courant de fond (12) qui comprend le solvant sélectif et qui est retourné à la seconde colonne de distillation extractive (K II).

8. Procédé selon l'une quelconque des revendications 5 à 7, **caractérisé en ce que** le liquide ou un courant partiel de liquide est prélevé de la seconde colonne de distillation extractive (K II) à partir d'un plateau théorique qui est situé un ou plusieurs plateaux théoriques en dessous du prélèvement latéral (10), est chauffé et/ou vaporisé par échange thermique indirect avec le courant de fond (13) provenant de la seconde colonne de distillation extractive (K II) et est retourné à la seconde colonne de distillation extractive (K II) sur le même plateau théorique ou au-dessus de celui-ci, le plateau à partir théorique duquel le liquide ou le courant partiel de liquide est prélevé étant choisi de sorte que l'exigence d'énergie pour la seconde colonne de distillation extractive (K II) soit minimale.
